# EUROPÄISCHE PATENTSCHRIFT

(11) **EP 4 273 877 B1**
(45) Veröffentlichungstag und Bekanntmachung des Hinweises auf die Patenterteilung: **29.10.2025**
(21) Anmeldenummer: 22171600.4
(22) Anmeldetag: 04.05.2022
(51) Int. Cl.: G16H 40/60

(54) **TRAGBARES SYSTEM SOWIE COMPUTERIMPLEMENTIERTES VERFAHREN ZUR UNTERSTÜTZUNG EINER PERSON MIT SEHEINSCHRÄNKUNGEN**
PORTABLE SYSTEM AND COMPUTER-IMPLEMENTED METHOD FOR SUPPORTING A PERSON WITH IMPAIRED VISION
SYSTÈME PORTABLE, AINSI QUE PROCÉDÉ MIS EN UVRE PAR ORDINATEUR PERMETTANT D'AIDER UNE PERSONNE AYANT DES TROUBLES VISUELS

(43) Veröffentlichungstag der Anmeldung: 08.11.2023
(73) Patentinhaber: DC Vision Systems GmbH, 90408 Nürnberg (DE)
(72) Erfinder: Lange, Frederik, 90408 Nürnberg (DE)
(74) Vertreter: Lösch, Christoph Ludwig Klaus

(56) Entgegenhaltungen:
- WO-A1-2017/202081
- WO-A2-2012/143952
- CN-A- 110 236 895
- CN-A- 110 664 593
- CN-A- 112 731 688
- US-A1- 2008 170 118
- US-A1- 2013 342 666

## Beschreibung

Die Erfindung betrifft ein tragbares System sowie ein computerimplementiertes Verfahren zur Unterstützung einer Person mit Seheinschränkungen.

Die Möglichkeit der visuellen Wahrnehmung ist für Menschen sehr wichtig. Entsprechend schwer ist es für Menschen mit einer Sehbehinderung bzw. Seheinschränkung am alltäglichen Leben vollumfänglich teilzunehmen.

US 2008/170118 A1 beschreibt ein tragbares System zur Unterstützung einer Person mit Seheinschränkungen. Das dortige System weist einen optischen Sensor, eine Recheneinheit und einem Aktuator auf. Es werden ein 3D-Modell der Umgebung erzeugt, Bewegungstrajektorien ermittelt und beispielsweise die Kollision der Person mit einem beweglichen Objekt vorhergesagt.

Der Erfindung liegt die Aufgabe zugrunde, ein System und ein Verfahren anzubieten, welches Personen mit Seheinschränkungen bei der Wahrnehmung ihrer Umgebung unterstützt.

Diese Aufgabe wird durch ein System mit den Merkmalen des Patentanspruchs 1 bzw. durch ein Verfahren mit den Merkmalen des Patentanspruchs 15 gelöst. Vorteilhafte Ausführungsformen sind in den Unteransprüchen beschrieben.

Erfindungsgemäß weist das tragbare System zur Unterstützung einer Person mit Seheinschränkungen mindestens einen optischen Sensor, mindestens einen Aktuator und eine Recheneinheit, die wirkfunktional mit dem Sensor und dem Aktuator in Verbindung steht, auf. Der optische Sensor wird am Kopf der Person getragen und ist ausgebildet, ein Umfeld der Person optisch zu erfassen. Der Aktuator ist ausgebildet, in Reaktion auf Steuersignale eine taktile und/oder akustische Rückmeldung an die Person zu geben. Die Recheneinheit ist ausgebildet, aus dem erfassten Umfeld ein dreidimensionales Umfeldmodell zu erzeugen, in dem Umfeldmodell zwischen beweglichen Objekten, unbeweglichen Objekten und Szenenbestandteilen zu unterscheiden, die beweglichen Objekte und die unbeweglichen Objekte zu klassifizieren, Bewegungstrajektorien der beweglichen Objekte relativ zu den unbeweglichen Objekten des Umfeldmodells zu bestimmen, die Position der Person und eine Bewegungstrajektorie der Person relativ zu den unbeweglichen Objekten des Umfeldmodells zu bestimmen, basierend auf einer Relativbewegung zwischen der Person einerseits und den beweglichen Objekten, den unbeweglichen Objekten und Szenenbestandteilen andererseits Interaktionen zu prädizieren, den Objekten ein Prioritätsattribut zuzuordnen, wobei entweder demjenigen Objekt, mit dem eine zeitlich nächstkommende Interaktion prädiziert wurde, das höchste Prioritätsattribut zugeordnet wird oder demjenigen Objekt, das sich in einer Blickrichtung der Person befinden ein höheres Prioritätsattribut zugeordnet wird als einem Objekt, das sich außerhalb der Blickrichtung befindet, und die Steuersignale für den Aktuator basierend auf den prädizierten Interaktionen für das Objekt bzw. die Objekte mit dem/den höchsten Prioritätsattribut/en zu erzeugen.

Das System erzeugt also aus dem Umfeld der Person abgeleitete taktile und/oder akustische Rückmeldungen, die von der Person mit Seheinschränkungen wahrgenommen werden können. Die Orientierungs-, Informations- und Interaktionsmöglichkeiten der Person mit Seheinschränkungen mit deren Umgebung werden dadurch verbessert.

Das System ist als tragbares System ausgebildet. Darunter wird verstanden, dass der optische Sensor, der Aktuator und die Recheneinheit derart ausgebildet sind, dass sie von der Person mit Seheinschränkungen getragen werden können. Es handelt sich also um ein mobiles System. In vorteilhafter Weise kann die Recheneinheit beispielsweise an einem Gürtel, mittels eines Brustgurtes oder eines Rucksackes getragen werden. Der Sensor kann beispielsweise in eine Kopfbedeckung, ein Stirnband oder ein Headset integriert sein. Der Aktuator kann ebenfalls in eine Kopfbedeckung, ein Stirnband oder ein Headset integriert sein oder auch in ein Armband. Um die Tragbarkeit des Systems zu gewährleisten, ist das System bzw. einzelne Komponenten des Systems bevorzugt mit einer oder mehreren netzunabhängigen Energiequellen (z.B. Akkumulatoren) ausgestattet.

Der mindestens eine optische Sensor dient hierbei zur optischen Erfassung des Umfeldes der Person mit Seheinschränkungen und wird von dieser am Kopf getragen. Der optische Sensor erfasst die zweidimensionale (2D) Bildinformation und die dreidimensionale (3D) Geometrie des Umfeldes. Der optische Sensor kann beispielsweise als 3D-Kamerasystem ausgebildet sein. Es erfolgt eine kontinuierliche Erfassung des Umfeldes, beispielsweise mit einer Bildfrequenz von 25 Bildern pro Sekunde. Es ist jedoch auch möglich ein LIDAR-Kamerasystem oder ähnliches in Kombination mit 2D-Bildsensoren einzusetzen.

Ferner ist es möglich, dass der optische Sensor ein vorbekannter 2D-Bildsensor ist. Durch die Bewegung des Kopfes kann der Sensor das Umfeld aus verschiedenen Perspektiven erfassen. Die 3D Information kann dann z. B. durch Korrelation der Bilddaten und Triangulation bestimmt werden. Falls mehrere 2D-Bildsensoren mit einem überlappenden Bildbereich und unterschiedlicher Position verwendet werden, kann die 3D Information auch z. B. durch Korrelation der Bilddaten und Triangulation bestimmt werden. Besonders vorteilhaft ist es in allen Sensor-Konfigurationen, die 3D Informationen aus mehreren einzelnen Messungen zu kombinieren und damit die Genauigkeit des Modells zu erhöhen.

Bei dem Umfeld, das durch den optischen Sensor dreidimensional erfasst wird, handelt es sich um die unmittelbare Umgebung (Nahfeld) der Person mit Seheinschränkungen, insbesondere die unmittelbare Umgebung bis zu einigen Metern Entfernung. Insbesondere ist der optische Sensor derart ausgebildet, dass durch diesen ein Zimmer, in dem sich die Person befindet, und die sich in diesem Zimmer befindenden Objekte erfasst werden können. Auch ist der optische Sensor derart ausgebildet, dass dieser im Freien etwa die Umgebung im Bereich von ca. 20 Metern um die Person mit Seheinschränkungen herum dreidimensional erfassen kann. Die 2D-Erfassung kann auch eine weitere Umgebung um die Person abdecken.

Der mindestens eine Aktuator gibt, in Reaktion auf entsprechende Steuersignale, die diesem von der Recheneinheit übermittelt werden, eine taktile und/oder akustische Rückmeldung an die Person mit Seheinschränkungen. Unter einer taktilen Rückmeldung wird hierbei jede Rückmeldung bzw. jeder Reiz verstanden, der über das Sinnesorgan der Haut von der Person mit Seheinschränkungen aufgenommen werden kann. Beispielsweise kann es sich bei diesem Reiz, der vom Aktuator erzeugt wird, um einen Druckreiz handeln. Es ist jedoch auch möglich, dass der Aktuator einen elektrischen Reiz erzeugt.

Die Recheneinheit ist wirkfunktional mit dem mindestens einen Sensor und dem mindestens einen Aktuator verbunden. Unter "wirkfunktional" wird hierbei jede Verbindung verstanden, die eine Kommunikation bzw. einen Signal- und/oder Datenaustausch zwischen der Recheneinheit und dem Sensor bzw. dem Aktuator ermöglicht. Diese Verbindung kann drahtgebunden, aber auch drahtlos (z.B. nach dem Bluetooth-, LTE- oder 5G-Standard) ausgeführt sein.

Die Recheneinheit ist als elektronische Recheneinheit ausgebildet, beispielsweise als Mikrocomputer oder Mikrocontroller mit entsprechenden Eingangs- und Ausgangsschnittstellen sowie internen Prozessor- und Speichereinheiten, sodass die Recheneinheit die vom optischen Sensor und weiteren Eingabegeräten zur Verfügung gestellten Daten empfangen und verarbeiten kann und als Ergebnis der Verarbeitung Daten an den Aktuator und weitere Ausgabegeräte ausgeben kann. Durch das Vorhandensein einer entsprechenden Programmierung (z.B. Softwaremodule) ist die Recheneinheit in der Lage (d.h. dafür ausgebildet), die im Folgenden beschriebenen Funktionalitäten auszuführen.

Die Recheneinheit ist hierbei derart ausgebildet, dass diese zunächst aus dem erfassten Umfeld ein dreidimensionales Umfeldmodell erzeugt. Hierbei kann es sich beispielsweise um ein voxelbasiertes Umfeldmodell handeln.

Außerdem ist die Recheneinheit derart ausgebildet, dass diese in dem Umfeldmodell zwischen beweglichen Objekten, unbeweglichen Objekten und Szenenbestandteilen unterscheiden kann. Unter beweglichen Objekten werden hierbei sowohl Objekte verstanden, die sich momentan bewegen, als auch Objekte, die sich ihrer Art nach bewegen können. Unter unbeweglichen Objekten werden Objekte verstanden, die sich weder momentan bewegen, noch ihrer Art nach bewegen können. Szenenbestandteile können beispielweise der Hintergrund des Umfelds oder sonstige Bestandteile der Umgebung sein, wie beispielsweise - je nach der vorliegenden Szene - Boden, Wände, Treppen, Grünflächen, Bürgersteig usw..

Ferner ist die Recheneinheit derart ausgebildet, dass diese zumindest die beweglichen Objekte und die unbeweglichen Objekte klassifiziert, d.h. bestimmten Klassen zuordnet. Die hierfür verwendeten Klassen können sich je nach Umfeld unterscheiden. Typische Klassen wären beispielsweise: Automobil, Fahrrad, Verkehrszeichen, Tisch, Stuhl, Tür, Trinkglas oder Unbekannt. Ein Objekt wird der Klasse Unbekannt zugeordnet, wenn keine Klassifikation in dem System bekannte Klassen erfolgen kann. Der Klassifikation kann auch eine Konfidenz zugeordnet werden. Mit dieser Konfidenz kann quantitativ dargestellt werden, wie sicher Objekte klassifiziert wurden.

Die Recheneinheit ist ferner dazu ausgebildet, Bewegungstrajektorien der beweglichen Objekte relativ zu den unbeweglichen Objekten bzw. Szenenbestandteilen des Umfeldmodells zu bestimmen, d.h. die Recheneinheit bestimmt die Bewegungsrichtung und -geschwindigkeit der einzelnen sich bewegenden Objekte.

Des Weiteren ist die Recheneinheit dazu ausgebildet, die Position der Person mit Seheinschränkungen innerhalb des Umfelds bzw. innerhalb des Umfeldmodells zu bestimmen und eine Bewegungstrajektorie der Person relativ zu den unbeweglichen Objekten und Szenenbestandteilen des Umfeldmodells zu bestimmen.

Daneben ist die Recheneinheit dazu ausgebildet, basierend auf einer Relativbewegung zwischen der Person mit Seheinschränkungen einerseits und den beweglichen Objekten, den unbeweglichen Objekten und Szenenbestandteilen andererseits Interaktionen zu prädizieren.

Unter Interaktionen werden hierbei beispielsweise Kollisionen der Person mit Seheinschränkungen mit beweglichen Objekten oder unbeweglichen Objekten verstanden. Darüber hinaus kann es sich bei Interaktionen jedoch beispielsweise auch um eine Gefahrenstelle im Umfeld handeln (z.B. Treppenstufe), auf die die Person mit Seheinschränkungen trifft. Ein weiteres Beispiel einer Interaktion wäre beispielsweise das Greifen der Person mit Seheinschränkungen nach einem Gegenstand im Umfeld.

Der Prädizierzeitraum, für den die Interaktionen prädiziert werden, ist abhängig vom vorliegenden Umfeld, liegt im Allgemeinen aber etwa zwischen 0,5 Sekunden und einigen Minuten, insbesondere zwischen 1 Sekunde und 1 Minute.

Schließlich ist die Recheneinheit dazu ausgebildet, basierend auf den prädizierten Interaktionen Steuersignale für den Aktuator zu erzeugen. Mit anderen Worten erzeugt die Recheneinheit je nach prädizierter Interaktion ein Steuersignal für den Aktuator, sodass der Aktuator in Reaktion auf diese Steuersignale eine taktile und/oder akustische Rückmeldung an die Person mit Seheinschränkungen gibt. Je nach Interaktion erhält die Person damit eine unterschiedliche taktile und/oder akustische Rückmeldung.

Auf diese Weise ist es durch das System möglich, die Person mit Seheinschränkungen durch situationsangepasste taktile und/oder akustische Rückmeldungen zu unterstützen.

Die Recheneinheit ist ferner ausgebildet, den Objekten ein Prioritätsattribut zuzuordnen und die Steuersignale für den Aktuator basierend auf den prädizierten Interaktionen für das Objekt bzw. die Objekte mit dem/den höchsten Prioritätsattribut/en zu erzeugen. Mit anderen Worten priorisiert die Recheneinheit die Objekte des Umfelds und erzeugt die Steuersignale des Aktuators für das oder die Objekte, die als am wichtigsten angesehen werden. Entweder wird dasjenige Objekt, mit dem die zeitlich nächstkommende Interaktion prädiziert wurde, mit dem höchsten Prioritätsattribut versehen oder das Prioritätsattribut in Abhängigkeit von der Blickrichtung der Person zu bestimmen. Insbesondere wird denjenigen Objekten, die sich in der Blickrichtung der Person befinden, eine höhere Priorität zugeordnet als Objekten, die sich außerhalb der Blickrichtung befinden.

In einer weiteren bevorzugten Ausführungsform kann die Recheneinheit ferner dazu ausgebildet sein, dem Umfeldmodell ein Szenenattribut zuzuordnen und die Steuersignale für den Aktuator zusätzlich basierend auf dem Szenenattribut zu erzeugen. Beispielsweise kann die Recheneinheit ermitteln, dass es sich bei dem momentan vorliegenden Umfeld um die Person mit Seheinschränkungen um einen Innenraum (Indoor) oder um eine Situation außerhalb eines Gebäudes (Outdoor) handelt. Entsprechend würde dem vorliegenden Umfeldmodell als Szenenattribut beispielsweise entweder "Indoor" oder "Outdoor" zugeordnet werden. Je nach Szenenattribut unterscheiden sich dann die vom Aktuator erzeugten Steuersignale. Auf diese Weise kann die Rückmeldung an die Person besonders situationsbedingt erfolgen. Die Szenenattribute lassen sich auch noch verfeinern und für weitere spezifischere Assistenzfunktionen des Systems verwenden. So kann es noch Szenenattribute für Geschäfte, Bahnhöfe oder ähnliches geben.

Zusätzlich zu dem mindestens einen optischen Sensor kann das System einen akustischen Sensor aufweisen, der das Umfeld der Person mit Seheinschränkungen akustisch erfasst. In diesem Fall berücksichtigt die Recheneinheit bei der Erzeugung des Umfeldmodells auch das vom akustischen Sensor erfasste akustische Umfeld. Zusätzlich kann auch bei der Erzeugung des Prioritätsattributs und/oder das Szenenattributs dieses akustische Umfeld berücksichtigt werden. Auf diese Weise ist eine noch genauere und zuverlässigere Modellierung des Umfeldes, des Prioritätsattributs und/oder des Szenenattributs möglich. Entsprechend exakter sind auch die erzeugten Steuersignale für den Aktuator und damit die Rückmeldung an die Person mit Seheinschränkungen.

Der taktile Aktuator kann als Stirndisplay ausgebildet sein, das beispielsweise in einem von der Person mit Seheinschränkungen getragenen Stirnband integriert ist. Dieses Stirndisplay ist derart ausgestaltet, dass das der Stirn der Person zugewandte Display an bestimmten Rasterpunkten Reize erzeugt, sodass sich für die Person mit Seheinschränkungen eine Information (Rückmeldung) ergibt. Der taktile Aktuator kann auch als Unterarmdisplay ausgebildet sein, das beispielsweise in einem von der Person mit Seheinschränkungen getragenes Armband integriert ist. Auch dieses Unterarmdisplay kann derart ausgestaltet sein, dass das dem Arm (v.a. dem Unterarm) zugewandte Display an bestimmten Rasterpunkten Reize erzeugt, sodass sich für die Person mit Seheinschränkungen eine Information ergibt.

Auch kann es sich bei dem Aktuator um einen Richtungsaktuator handeln, der in Reaktion auf die Steuersignale der Recheneinheit ein bewegliches Reaktionselement in eine vorbestimmte Richtung bewegt. Diese Bewegung wiederum kann von der Person mit Seheinschränkungen beispielsweise als Druckreiz wahrgenommen werden, wenn diese Person ein Armband trägt, in dem ein derartiger Richtungsaktuator integriert ist, oder wenn diese Person den Richtungsaktuator anderweitig berührt. Die Ausgestaltung des Aktuator als Richtungsaktuator ist insbesondere dann vorteilhaft, wenn als prädizierte Interaktion eine Greifbewegung der Person vorliegt. Durch entsprechende Rückmeldungen über den Richtungsaktuator an die Person kann die Greifbewegung (z.B. die Position der Hand der Person) beeinflusst, korrigiert und unterstützt werden.

Des Weiteren kann der Aktuator derart ausgebildet sein, dass Informationen über das Umfeld der Person mit Seheinschränkungen weitergeleitet werden.

In einer bevorzugten Ausführungsform kann die Recheneinheit dazu ausgebildet sein, die Unterscheidung der Objekte, die Klassifizierung der Objekte, das Prädizieren der Interaktionen und/oder die Erzeugung der Steuersignale für den Aktuator mit Hilfe eines oder mehrerer Verfahren zum maschinellen Lernen, insbesondere unter Verwendung von sogenannten Neuronalen Netzen, durchzuführen.

In einer weiteren bevorzugten Ausführungsform kann die Recheneinheit dazu ausgebildet sein, für Objekte, die in derselben Objektklasse klassifiziert sind, gleiche Steuersignale zu erzeugen. So kann beispielsweise für jedes Objekt, das der Klasse "Automobil" zugeordnet wurde, das gleiche Symbol (z.B. stilisierte Silhouette eines Automobils oder ein Punkt-Strich-Muster) durch den Aktuator abgebildet werden. Auf diese Weise kann die Person mit Seheinschränkung ohne großen kognitiven Aufwand rasch erkennen, um welches Objekt es sich handelt.

Die Komplexität und die Auflösung der an die Person mit Seheinschränkungen übermittelbaren Symbole sind im Allgemeinen auf relativ wenige gleichzeitig von der Person spürbare und vom Aktuator erzeugbare Rasterpunkte beschränkt. Entsprechend werden entweder sehr vereinfachte und abstrakte bildliche Symbole dargestellt oder es wird ein Punkt-Strich-Muster dargestellt, dessen Bedeutungsgehalt die Person mit Seheinschränkungen dann ggf. lernen muss.

Sowohl die bildlichen Symbole als auch die Punkt-Strich-Muster können als Sequenzen (d.h. zeitlich veränderlich) dargestellt werden. Insbesondere kann das Symbol oder das Punkt-Strich-Muster in Art eines Laufbandes, pulsierend und/oder mit sich verändernder Größe dargestellt werden. Auf diese Weise kann der Informationsgehalt ohne die Notwendigkeit einer höheren Auflösung an Rasterpunkten vergrößert werden.

**In** einer weiteren bevorzugten Ausführungsform weist das System ferner eine Fernzugriffseinheit auf. Diese Fernzugriffseinheit ist ausgebildet, auf das Umfeldmodell zuzugreifen und dieses ggf. anzupassen oder zu verändern. Außerdem ist die Fernzugriffseinheit dazu ausgebildet, mit der Person mit Seheinschränkungen zu kommunizieren und/oder Steuersignale für den Aktuator zu erzeugen. Die Fernzugriffseinheit ist bevorzugterweise über eine drahtlose Kommunikationsverbindung mit der Recheneinheit verbunden.

**In** einer weiteren bevorzugten Ausführungsform kann die Recheneinheit ferner dazu ausgebildet sein, basierend auf einer gewünschten Zielposition der Person mit Seheinschränkungen und dem Umfeldmodell eine Pfadvorgabe zum Erreichen der Zielposition zu bestimmen und basierend auf der Pfadvorgabe Steuersignale für den Aktuator zu erzeugen. Auf diese Weise kann das System als Navigationshilfe dienen und dadurch die Person mit Seheinschränkungen noch mehr unterstützen.

Das erfindungsgemäße computerimplementierte Verfahren zur Unterstützung einer Person mit Seheinschränkungen weist folgende Verfahrensschritte auf: (1) Erfassen eines Umfelds der Person mittels eines optischen Sensors; (2) Erzeugen eines dreidimensionale Umfeldmodells; (3) Unterscheiden zwischen beweglichen Objekten, unbeweglichen Objekten und Szenenbestandteilen in dem Umfeldmodell; (4) Klassifizieren der beweglichen Objekte und der unbeweglichen Objekte; (5) Bestimmen von Bewegungstrajektorien der beweglichen Objekte relativ zu den unbeweglichen Objekten des Umfeldmodells; (6) Bestimmen der Position und Bewegungstrajektorie der Person relativ zu den unbeweglichen Objekten des Umfeldmodells; (7) Prädizieren von Interaktionen basierend auf einer Relativbewegung zwischen der Person einerseits und den beweglichen Objekten, den unbeweglichen Objekten und Szenenbestandteilen andererseits; (8) Zuordnen eines Prioritätsattributs zu den Objekten, wobei entweder demjenigen Objekt, mit dem eine zeitlich nächstkommende Interaktion prädiziert wurde, das höchste Prioritätsattribut zugeordnet wird oder demjenigen Objekt, das sich in einer Blickrichtung der Person (P) befinden ein höheres Prioritätsattribut zugeordnet wird als einem Objekt, das sich außerhalb der Blickrichtung befindet; und (9) Erzeugen von Steuersignalen für einen taktilen und/oder akustischen Aktuator, basierend auf den prädizierten Interaktionen für das Objekt bzw. die Objekte mit dem/den höchsten Prioritätsattribut/en.

Die Erfindung ist anhand eines Ausführungsbeispiels in den Zeichnungsfiguren weiter erläutert, wobei gleiche Bezugszeichen gleiche bzw. gleichwirkende Komponenten bezeichnen. Es zeigen:
- Fig. 1: eine schematische Darstellung eines tragbaren Systems zur Unterstützung einer Person mit Seheinschränkungen;
- Fig. 2: ein schematische Blockschaubild des Systems aus Fig. 1; und
- Fig. 3: ein Ablaufdiagramm von Teilschritten eines Verfahrens zur Unterstützung einer Person mit Seheinschränkungen, welches von dem System aus Fig. 1 ausgeführt wird.

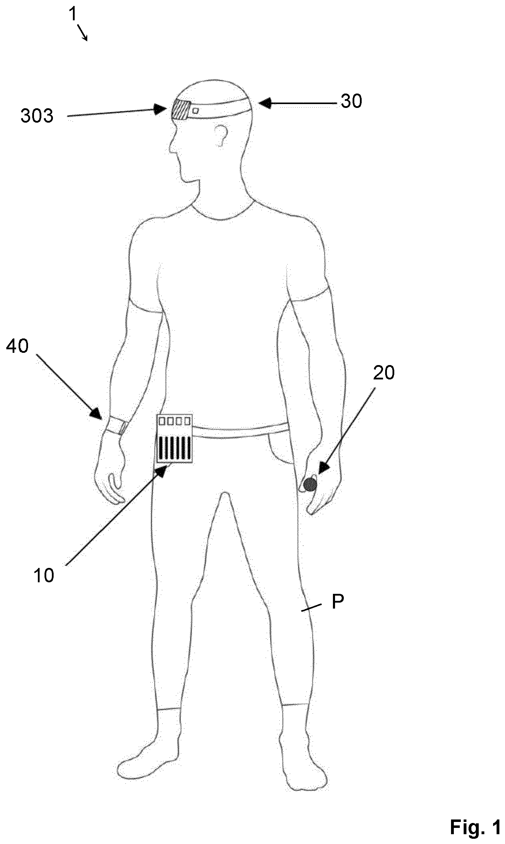

Fig. 1 zeigt schematisch ein tragbares System 1 zur Unterstützung einer Person P mit Seheinschränkungen. Das System 1 besteht aus den Hauptkomponenten Recheneinheit 10, Bediencontroller 20, Stirnband 30, Armband 40 und - in Fig. 1 nicht dargestellt - Fernzugriffseinheit 50.

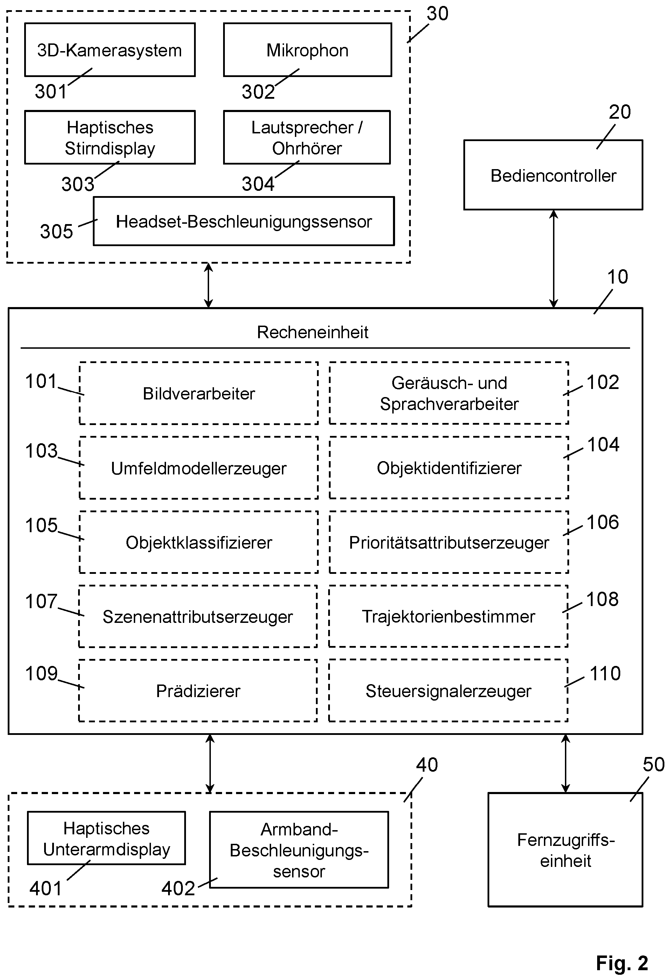

Das System 1 wird nun unter Bezugnahme auf Fig. 1 und Fig. 2 näher beschrieben.

Die Recheneinheit 10 ist als tragbarer Microcomputer ausgebildet und kann von der Person P beispielsweise an einem Gürtel, Tragegurt oder in einem Rucksack getragen werden. Die Recheneinheit 10 ist mit Softwaremodulen versehen, die als Bildverarbeiter 101, Geräusch- und Sprachverarbeiter 102, Umfeldmodellerzeuger 103, Objektidentifizierer 104, Objektklassifizierer 105, Prioritätsattributserzeuger 106, Szenenattributserzeuger 107, Trajektorienbestimmer 108, Prädizierer 109 und Steuersignalerzeuger 110 fungieren. Durch das Ausführen dieser Softwaremodule kann die Recheneinheit 10 die jeweiligen im Folgenden noch näher beschriebenen Funktionalitäten ausführen. Die Softwaremodule können hierbei als einzelne, voneinander getrennt vorliegende Module ausgestaltet sein. Es ist jedoch auch möglich, mehrere oder sämtliche Module in ein gemeinsames Modul zu integrieren. Beispielsweise können der Objektidentifizierer 104 und der Objektklassifizierer 105 in einem gemeinsamen Objektidentifizier- und Objektklassifiziermodul zusammengefasst sein.

Der Bediencontroller 20 ist wirkfunktional mit der Recheneinheit 10 verbunden und dient der Steuerung der Recheneinheit 10 durch die Person P oder einer weiteren Person. Hierfür besitzt der Bediencontroller 20 eine oder mehrere Mensch-Maschine-Schnittstellen.

Das Stirnband 30 wird von der Person P am Kopf getragen und fungiert als Headset. **In** das Stirnband 30 integriert sind ein 3D-Kamerasystem 301, ein Mikrophon 302, ein haptisches Stirndisplay 303, ein Lautsprecher 304 sowie ein Headset-Beschleunigungssensor 305. Diese Komponenten des Stirnbands 30 sind wirkfunktional mit der Recheneinheit 10 verbunden. Das 3D-Kamerasystem erfasst optisch das Umfeld der Person P und stellt einen "optischen Sensor" im Sinn der vorliegenden Erfindung dar. Das Mikrophon 302 erfasst akustisch das Umfeld der Person P und stellt einen "akustischen Sensor" im Sinn der vorliegenden Erfindung dar. Das haptische Stirndisplay 303 ist derart ausgestaltet, dass es in Reaktion auf Steuersignale der Recheneinheit 10 auf einem der Stirn der Person P zugewandten Display an bestimmten Rasterpunkten taktile Reize erzeugt. Das haptische Stirndisplay 303 stellt somit einen "Aktuator, der ausgebildet ist, in Reaktion auf Steuersignale eine taktile Rückmeldung an die Person zu geben" dar. Der Lautsprecher 304 ist in einen Ohrhörer integriert und ist derart ausgestaltet, dass er in Reaktion auf Steuersignale der Recheneinheit 10 akustische Signale erzeugt. Der Lautsprecher 304 stellt damit einen "Aktuator, der ausgebildet ist, in Reaktion auf Steuersignale eine akustische Rückmeldung an die Person zu geben" dar. Die vom Headset-Beschleunigungssensor 305 ermittelten Daten werden zusammen mit den Daten des 3D-Kamerasystems von der Recheneinheit 10 zur Bestimmung der Position der Person P und der Bewegung der Person P relativ zu dessen Umfeld verwendet.

In das Armband 40 ist ein haptisches Unterarmdisplay 401 und ein Armband-Beschleunigungssensor 402 integriert. Diese Komponenten des Armbands 40 sind wirkfunktional mit der Recheneinheit 10 verbunden. Das haptische Unterarmdisplay 401 ist derart ausgestaltet, dass es in Reaktion auf Steuersignale der Recheneinheit 10 auf einem dem Unterarm der Person P zugewandten Display an bestimmten Rasterpunkten taktile Reize erzeugt. Das haptische Unterarmdisplay 303 stellt somit einen weiteren "Aktuator, der ausgebildet ist, in Reaktion auf Steuersignale eine taktile Rückmeldung an die Person zu geben" dar. Die vom Armband-Beschleunigungssensor 402 ermittelten Daten werden zusammen mit den Daten des 3D-Kamerasystems von der Recheneinheit 10 zur Bestimmung der Position der Person P und der Bewegung der Person P relativ zu dessen Umfeld und/oder zur Bestimmung der Position des Armes / der Hand der Person P verwendet.

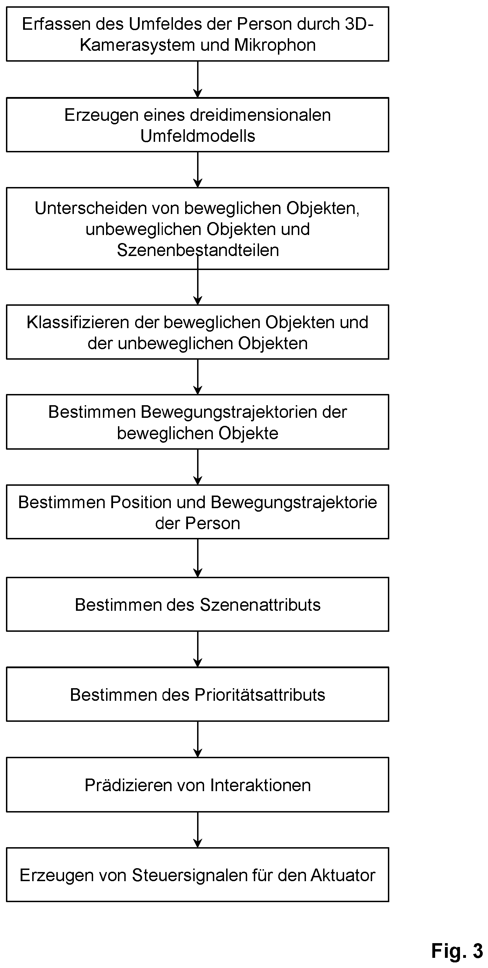

Das durch das System 1 durchgeführte computerimplementierte Verfahren zur Unterstützung der Person P ist in Fig. 3 mit Hilfe eines Ablaufdiagramms dargestellt.

Zunächst erfassen das 3D-Kamerasystem 301 und das Mikrophon 302 das Umfeld der Person P. Genauer erfasst das 3D-Kamerasystem 301 das Umfeld optisch und das Mikrophon 302 erfasst das Umfeld akustisch. Die vom 3D-Kamerasystem 301 erfassten optischen Daten werden durch den Bildverarbeiter 101 aufbereitet. Der Geräusch- und Sprachverarbeiter 102 wiederum bereitet die vom Mikrophon 302 erfassten akustischen Daten auf.

Der Umfeldmodellerzeuger 103 erzeugt aus den aufbereiteten optischen und akustischen Daten ein dreidimensionales Umfeldmodell. Der Objektidentifizierer 104 unterscheidet in dem dreidimensionalen Umfeldmodell bewegliche Objekte, unbewegliche Objekte und Szenenbestandteile voneinander. Der Objektklassifizierer 105 klassifiziert die beweglichen Objekte und die unbeweglichen Objekte, d.h. der Objektklassifizierer 105 ordnet die erkannten beweglichen Objekte und unbeweglichen Objekte verschiedenen Objektklassen zu.

Der Trajektorienbestimmer 108 bestimmt die Bewegungstrajektorien der beweglichen Objekte relativ zu den unbeweglichen Objekten des Umfeldmodells. Der Prädizierer 109 prädiziert, basierend auf einer Relativbewegung zwischen der Person P einerseits und den beweglichen Objekten, den unbeweglichen Objekten und Szenenbestandteilen andererseits, Interaktionen. Der Steuersignalerzeuger 110 erzeugt, basierend auf den prädizierten Interaktionen, Steuersignale für das haptische Stirndisplay 303, den Lautsprecher 304 und/oder das haptische Unterarmdisplay 401.

Der Prioritätsattributserzeuger 106 ordnet den Objekten ein Prioritätsattribut zu. Der Szenenattributserzeuger 107 ordnet dem Umfeldmodell ein Szenenattribut zu.

Je nach erfasstem Umfeld und vorliegender Szene unterstützt das System 1 die Person P auf unterschiedliche Weise. Folgende Anwendungsbeispiele sind exemplarisch beschrieben:

### Anwendungsbeispiel 1: Orientierung im Straßenverkehr

Befindet sich die Person P in einem Straßenverkehrsumfeld, so erfasst das 3D-Kammerasystem 301 dieses Umfeld optisch und das Mikrophon 302 erfasst dieses Umfeld akustisch. Aus den erfassten Daten erzeugt der Umfeldmodellerzeuger 103 ein dreidimensionales Umfeldmodell. Der Objekteidentifizierer 104 identifiziert Objekte dieses Umfelds, z.B. bewegliche Objekte wie verschiedene Automobile, Fahrräder, Lastkraftwägen, Busse, Personen oder unbewegliche Objekte wie verschiedene Verkehrszeichen, Ampeln, Gehwege, Bordstein, Tische, Stühle, Abfallbehälter. Der Szenenattributserzeuger 107 erzeugt, basierend auf den identifizierten Objekten und Geräuschen sowie basierend ggf. auf weiteren Informationen (z.B. GPS-Daten), ein Szenenattribut, im vorliegenden Fall das Attribut "Outdoor".

Der Trajektorienbestimmer 108 bestimmt für die beweglichen Objekte eine Bewegungstrajektorie und der Prädizierer 109 prädiziert, basierend auf der Relativbewegung zwischen der Person P einerseits und den beweglichen Objekten, den unbeweglichen Objekten und Szenenbestandteilen andererseits Interaktionen. Die Position der Person P sowie deren Bewegungstrajektorie wird hierbei basierend auf Daten des Headset-Beschleunigungssensors 305 und/oder des Armband-Beschleunigungssensors 402 und dem 3D-Kamerasystem 303 ermittelt.

Beispielsweise könnte der Prädizierer 109 im Ergebnis prädizieren, dass es bei gleichbleibender Relativbewegung der Person P in 20 Sekunden zu einer Kollision mit einem unbeweglichen Objekt (z.B. mit einer auf dem Gehweg stehenden Mülltonne) und in 10 Sekunden zu einer Kollision mit einem beweglichen Objekt (z.B. einer weiteren Person) kommt. Der Prioritätsattributserzeuger 106 weist nun dem beweglichen Objekt ein höheres Prioritätsattribut zu, da diese Interaktion zeitlich vor der Interaktion mit dem unbeweglichen Objekt stattfinden würde.

Der Steuersignalerzeuger 110 erzeugt nun, basierend auf der Interaktion bzw. dem Objekt mit dem höchsten Prioritätsattribut und unter Berücksichtigung des Szenenattributs "Outdoor", Steuersignale für das haptische Stirndisplay 303 und das haptische Unterarmdisplay 401. Genauer erzeugt der Steuersignalerzeuger 110 Steuersignale, die auf dem Stirndisplay 303 und dem Unterarmdisplay 401 oder einem anderen taktilen Signalgeber Reize in Form eines Punkt-Strich-Musters erzeugen. Bei einer genügend hohen Auflösung kann alternativ auch eine stilisierte menschliche Silhouette erzeugt werden. Auf diese Weise erhält die Person P ein taktile Rückmeldung, dass eine Kollision mit einer Person prädiziert wird, und kann sein Verhalten entsprechend ändern (z.B. stehen bleiben oder Bewegungsrichtung ändern).

### Anwendungsbeispiel 2: Orientierung in einem Gebäude

Befindet sich die Person P in einem Gebäude bzw. in einem Raum innerhalb des Gebäudes, so erfasst das 3D-Kammerasystem 301 dieses Umfeld wieder optisch und das Mikrophon 302 erfasst dieses Umfeld wieder akustisch. Aus den erfassten Daten erzeugt der Umfeldmodellerzeuger 103 wieder ein dreidimensionales Umfeldmodell. Der Objekteidentifizierer 104 identifiziert Objekte dieses Umfelds, z.B. bewegliche Objekte wie Personen oder unbewegliche Objekte wie verschiedene Tische, Stühle, Türen, Trinkgläser, Stufen. Der Szenenattributserzeuger 107 erzeugt, basierend auf den identifizierten Objekten und Geräuschen und ggf. basierend auf weiteren Informationen, ein Szenenattribut, im vorliegenden Fall das Attribut "Indoor".

Der Trajektorienbestimmer 108 bestimmt für die beweglichen Objekte eine Bewegungstrajektorie und der Prädizierer 109 prädiziert, basierend auf der Relativbewegung zwischen der Person P einerseits und den beweglichen Objekten, den unbeweglichen Objekten und Szenenbestandteilen andererseits Interaktionen. Beispielsweise könnte der Prädizierer 109 im Ergebnis prädizieren, dass es bei gleichbleibender Relativbewegung der Person P in 10 Sekunden zu einer Kollision mit einer geschlossenen Tür kommt und die Person P bei gleichbleibender Relativbewegung in 5 Sekunden eine (nach unten führende) Stufe erreicht. Der Prioritätsattributserzeuger 106 weist nun der Stufe ein höheres Prioritätsattribut zu, da diese Interaktion zeitlich vor der Interaktion mit der geschlossenen Tür stattfinden würde.

Der Steuersignalerzeuger 110 erzeugt nun, basierend auf der Interaktion bzw. dem Objekt mit dem höchsten Prioritätsattribut und unter Berücksichtigung des Szenenattributs "Indoor", Steuersignale für das haptische Stirndisplay 303 und den Lautsprecher 304. Genauer erzeugt der Steuersignalerzeuger 110 Steuersignale, die auf dem Stirndisplay 303 Reize in Form eines Punkt-Strich-Musters erzeugen. Bei einer genügend hohen Auflösung kann alternativ auch eine stilisierte Silhouette einer nach unten führenden Stufe erzeugen werden. Außerdem erzeugt der Steuersignalerzeuger 110 Steuersignale, die dazu führen, dass der Lautsprecher 304 einen Warnhinweis, wie "Achtung, Stufe nach unten" ausgibt. Auf diese Weise erhält die Person P ein taktile und eine akustische Rückmeldung, dass eine nach unten führende Stufe prädiziert wird, und kann sein Verhalten daran anpassen.

### Anwendungsbeispiel 3: Unterstützung bei Greifbewegung

Befindet sich die Person P beispielsweise vor einem Tisch, auf dem ein Trinkglas steht und möchte sie dieses Trinkglas greifen, so erfasst das 3D-Kammerasystem 301 das Umfeld wieder optisch und das Mikrophon 302 erfasst dieses Umfeld wieder akustisch. Aus den erfassten Daten erzeugt der Umfeldmodellerzeuger 103 wieder ein dreidimensionales Umfeldmodell. Der Objekteidentifizierer 104 identifiziert Objekte dieses Umfelds, insbesondere das Trinkglas.

Der Trajektorienbestimmer 108 bestimmt die Relativbewegung der Person P - genauer die Position und die Bewegung einer aus den Daten des Armband-Beschleunigungssensors 402 abgeleiteten Hand der Person P - relativ zum Trinkglas. Je nach Abstand und räumlicher Lage zwischen dem Trinkglas und der Hand der Person **P,** prädiziert der Prädizierer 109, wann das Trinkglas gegriffen wird.

Der Steuersignalerzeuger 110 erzeugt dann, basierend auf dem Abstand und der räumlicher Lage zwischen dem Trinkglas und der Hand der Person **P,** Steuersignale für das Armband 40. Genauer erzeugt der Steuersignalerzeuger 110 Steuersignale, die auf dem Unterarmdisplay 401 Reize in Form einer Richtungsangabe erzeugen (z.B. stilisierter Pfeil). Auf diese Weise erhält die Person P eine taktile Rückmeldung dahingehend, in welche Richtung er seine Hand bewegen muss, um das Trinkglas greifen zu können.

Ist das System 1 mit einem Richtungsaktuator (in den Figuren nicht dargestellt) ausgestattet, so kann der Steuersignalerzeuger 110 alternativ oder zusätzlich Steuersignale erzeugen, die das bewegliches Reaktionselement des Richtungsaktuators in eine bestimmte Richtung bewegen. So erhält die Person P eine taktile Rückmeldung dahingehend, in welche Richtung er seine Hand bewegen muss, um das Trinkglas greifen zu können.

### BEZUGSZEICHENLISTE

1 System
10 Recheneinheit
20 Bediencontroller
30 Headset
40 Armband
50 Fernzugriffseinheit
101 Bildverarbeiter
102 Geräusch- und Sprachverarbeiter
103 Umfeldmodellerzeuger
104 Objektidentifizierer
105 Objektklassifizierer
106 Prioritätsattributserzeuger
107 Szenenattributserzeuger
108 Trajektorienbestimmer
109 Prädizierer
110 Steuersignalerzeuger
301 3D-Kamerasystem
302 Mikrophon
303 Haptisches Stirndisplay
304 Lautsprecher/Ohrhörer
305 Headset-Beschleunigungssensor
401 Haptisches Unterarmdisplay
402 Armband-Beschleunigungssensor
P Person

## Patentansprüche

1. Tragbares System (1) zur Unterstützung einer Person (P) mit Seheinschränkungen, aufweisend:
- mindestens einen optischen Sensor (301), der am Kopf der Person (P) getragen wird und der ausgebildet ist, ein Umfeld der Person (P) optisch zu erfassen;
- mindestens einen Aktuator (303, 304, 401), der ausgebildet ist, in Reaktion auf Steuersignale eine taktile und/oder akustische Rückmeldung an die Person (P) zu geben;
- eine Recheneinheit (10), die wirkfunktional mit dem Sensor (301) und dem Aktuator (303, 304, 401) in Verbindung steht und die ausgebildet ist:
o aus dem erfassten Umfeld ein dreidimensionales Umfeldmodell zu erzeugen;
o in dem Umfeldmodell zwischen beweglichen Objekten, unbeweglichen Objekten und Szenenbestandteilen zu unterscheiden;
o die beweglichen Objekte und die unbeweglichen Objekte zu klassifizieren;
o Bewegungstrajektorien der beweglichen Objekte relativ zu den unbeweglichen Objekten des Umfeldmodells zu bestimmen;
o die Position der Person (P) und eine Bewegungstrajektorie der Person relativ zu den unbeweglichen Objekten des Umfeldmodells zu bestimmen; und
o basierend auf einer Relativbewegung zwischen der Person (P) einerseits und den beweglichen Objekten, den unbeweglichen Objekten und Szenenbestandteilen andererseits Interaktionen zu prädizieren;
**dadurch gekennzeichnet** die Recheneinheit (10) ferner ausgebildet ist:
o den Objekten ein Prioritätsattribut zuzuordnen, wobei entweder demjenigen Objekt, mit dem eine zeitlich nächstkommende Interaktion prädiziert wurde, das höchste Prioritätsattribut zugeordnet wird oder demjenigen Objekt, das sich in einer Blickrichtung der Person (P) befinden ein höheres Prioritätsattribut zugeordnet wird als einem Objekt, das sich außerhalb der Blickrichtung befindet; und
o die Steuersignale für den Aktuator (303, 304, 401) basierend auf den prädizierten Interaktionen für das Objekt bzw. die Objekte mit dem/den höchsten Prioritätsattribut/en zu erzeugen.

2. System nach einem der vorhergehenden Ansprüche, wobei die Recheneinheit (10) ferner ausgebildet ist:
o dem Umfeldmodell ein Szenenattribut zuzuordnen; und
o die Steuersignale für den Aktuator (303, 304, 401) zusätzlich basierend auf dem Szenenattribut zu erzeugen.

3. System nach einem der vorhergehenden Ansprüche ferner aufweisend einen akustischen Sensor (302), der ausgebildet ist, das Umfeld der Person (P) akustisch zu erfassen, und
wobei die Recheneinheit (10) ausgebildet ist, das Umfeldmodell, das Prioritätsattribut und, falls rückbezogen auf den Anspruch 2, das Szenenattribut aus dem vom optischen Sensor (301) und dem akustischen Sensor (302) erfassten Umfeld zu erzeugen.

4. System nach einem der vorhergehenden Ansprüche aufweisend mindestens einen taktilen Aktuator (303, 401) und mindestens einen akustischen Aktuator (304), wobei die Recheneinheit (10) ausgebildet ist, in Abhängigkeit von prädizierten Interaktionen Steuersignale für den taktilen Aktuator (303, 401) und/oder den akustischen Aktuator (304) zu erzeugen.

5. System nach einem der vorhergehenden Ansprüche aufweisend einen ersten taktilen Aktuator, der als ein in einem von der Person (P) tragbaren Stirnband (30) integriertes Stirndisplay (303) ausgebildet ist, und einen zweiten taktilen Aktuator, der als ein in einem von der Person tragbaren Armband (40) integriertes Unterarmdisplay (401) und/oder als ein in das tragbare Armband (40) integrierter Richtungsaktuator ausgebildet ist.

6. System nach Anspruch 5, wobei die Recheneinheit (10) ausgebildet ist, die Steuersignale für das Unterarmdisplay (401) und/oder den Richtungsaktuator basierend auf einer Entfernung und/oder einer räumlichen Lage zwischen einem der Objekte einerseits und dem Armband (40), einem Arm der Person (P) oder einer Hand der Person (P) andererseits zu erzeugen.

7. System nach einem der vorhergehenden Ansprüche, wobei die Recheneinheit (10) ausgebildet ist, die Unterscheidung der Objekte, die Klassifizierung der Objekte, das Prädizieren der Interaktionen und/oder die Erzeugung der Steuersignale für den Aktuator (303, 304, 401) mit Hilfe eines oder mehrerer Verfahren zum maschinellen Lernen durchzuführen.

8. System nach einem der vorhergehenden Ansprüche, wobei die Recheneinheit (10) ausgebildet ist, für Objekte, die in derselben Objektklasse klassifiziert sind, gleiche Steuersignale zu erzeugen.

9. System nach einem der vorhergehenden Ansprüche, wobei der optische Sensor (301) als 3D-Kamerasystem, insbesondere als ein in einem von der Person tragbares Headset (30) integriertes 3D-Kamerasystem, ausgebildet ist.

10. System nach einem der vorhergehenden Ansprüche ferner aufweisend eine Fernzugriffseinheit (50), welche ausgebildet ist, auf das Umfeldmodell zuzugreifen, mit der Person (P) zu kommunizieren und/oder Steuersignale für den Aktuator (303, 304, 401) zu erzeugen.

11. System nach einem der vorhergehenden Ansprüche, wobei über den Aktuator (303, 304, 401) Informationen über das Umfeld der Person (P) übermittelt werden.

12. System nach einem der vorhergehenden Ansprüche, wobei die Recheneinheit ferner ausgebildet ist, basierend auf einer Zielposition der Person (P) und dem Umfeldmodell eine Pfadvorgabe zum Erreichen der Zielposition zu bestimmen und basierend auf der Pfadvorgabe Steuersignale für den Aktuator (303, 304, 401) zu erzeugen.

13. System nach Anspruch 12, wobei die Zielposition aus einem Navigationssystem, insbesondere aus einem Fußgängernavigationssystem, stammt.

14. System nach einem der vorhergehenden Ansprüche, wobei die Recheneinheit ferner ausgebildet ist, bei der Klassifizierung der beweglichen Objekte und der unbeweglichen Objekte eine Konfidenz für die beweglichen Objekten und die unbeweglichen Objekte zu bestimmen.

15. Computerimplementiertes Verfahren zur Unterstützung einer Person (P) mit Seheinschränkungen mit folgenden Verfahrensschritten:
- Erfassen eines Umfelds der Person (P) mittels eines optischen Sensors (301);
- Erzeugen eines dreidimensionale Umfeldmodells;
- Unterscheiden zwischen beweglichen Objekten, unbeweglichen Objekten und Szenenbestandteilen in dem Umfeldmodell;
- Klassifizieren der beweglichen Objekte und der unbeweglichen Objekte;
- Bestimmen von Bewegungstrajektorien der beweglichen Objekte relativ zu den unbeweglichen Objekten des Umfeldmodells;
- Bestimmen der Position und Bewegungstrajektorie der Person (P) relativ zu den unbeweglichen Objekten des Umfeldmodells;
- Prädizieren von Interaktionen basierend auf einer Relativbewegung zwischen der Person (P) einerseits und den beweglichen Objekten, den unbeweglichen Objekten und Szenenbestandteilen andererseits;
- **dadurch gekennzeichnet, dass** ein Prioritätsattribut den Objekten zugeordnet wird wobei entweder demjenigen Objekt, mit dem eine zeitlich nächstkommende Interaktion prädiziert wurde, das höchste Prioritätsattribut zugeordnet wird oder demjenigen Objekt, das sich in einer Blickrichtung der Person (P) befinden ein höheres Prioritätsattribut zugeordnet wird als einem Objekt, das sich außerhalb der Blickrichtung befindet; und
- ein Steuersignal für einen taktilen und/oder akustischen Aktuator (303, 304, 401), basierend auf den prädizierten Interaktionen für das Objekt bzw. die Objekte mit dem/den höchsten Prioritätsattribut/en. erzeugt wird

## Claims

1. Portable system (1) for assisting a person (P) with visual impairments, comprising:
- at least one optical sensor (301) that is worn on the head of the person (P) and designed to optically capture surroundings of the person (P);
- at least one actuator (303, 304, 401) that is designed to provide tactile and/or acoustic feedback to the person (P) in response to control signals;
- a computing unit (10) that is operatively connected to the sensor (301) and the actuator (303, 304, 401) and designed:
∘ to generate a three-dimensional model of the surroundings from the captured surroundings;
∘ to distinguish between moving objects, stationary objects and scene elements in the model of the surroundings;
∘ to classify the moving objects and the stationary objects;
∘ to determine the movement trajectories of the moving objects relative to the stationary objects in the model of the surroundings;
∘ to determine the position of the person (P) and a movement trajectory of the person relative to the stationary objects in the model of the surroundings; and
∘ to predict interactions on the basis of a relative movement between firstly the person (P) and secondly the moving objects, the stationary objects and scene elements;
**characterized in that** the computing unit (10) is further designed:
o to assign a priority attribute to the objects, wherein either the highest priority attribute is assigned to the object predicted to have the next upcoming interaction or the object that is within a line of sight of the person (P) is assigned a higher priority attribute than an object that is away from the line of sight; and
∘ to generate the control signals for the actuator (303, 304, 401) on the basis of the predicted interactions for the object(s) with the highest priority attribute (s) .

2. System according to any of the preceding claims, wherein the computing unit (10) is further designed:
o to assign a scene attribute to the model of the surroundings; and
∘ to generate the control signals for the actuator (303, 304, 401) on the basis of the scene attribute as well.

3. System according to any of the preceding claims, further comprising an acoustic sensor (302) that is designed to acoustically record the surroundings of the person (P), and
wherein the computing unit (10) is designed to generate the model of the surroundings, the priority attribute and, if referring back on Claim 2, the scene attribute from the surroundings captured by the optical sensor (301) and recorded by the acoustic sensor (302).

4. System according to any of the preceding claims, comprising at least one tactile actuator (303, 401) and at least one acoustic actuator (304), wherein the computing unit (10) is designed to generate control signals for the tactile actuator (303, 401) and/or the acoustic actuator (304) in a manner dependent on predicted interactions.

5. System according to any of the preceding claims, comprising a first tactile actuator in the form of a forehead display (303) which is integrated in a headband (30) that can be worn by the person (P) and a second tactile actuator in the form of a forearm display (401) which is integrated in a bracelet (40) that can be worn by the person and/or in the form of a direction actuator which is integrated in the wearable bracelet (40).

6. System according to Claim 5, wherein the computing unit (10) is designed to generate the control signals for the forearm display (401) and/or the direction actuator on the basis of a distance and/or a spatial position between firstly one of the objects and secondly the bracelet (40), an arm of the person (P) or a hand of the person (P).

7. System according to any of the preceding claims, wherein the computing unit (10) is designed to perform the differentiation of the objects, the classification of the objects, the prediction of the interactions and/or the generation of the control signals for the actuator (303, 304, 401) with the aid of one or more machine learning methods.

8. System according to any of the preceding claims, wherein the computing unit (10) is designed to generate the same control signals for objects that are classified in the same object class.

9. System according to any of the preceding claims, wherein the optical sensor (301) is in the form of a 3-D camera system, in particular a 3-D camera system which is integrated in a headset (30) that can be worn by the person.

10. System according to any of the preceding claims, further comprising a remote access unit (50) that is designed to access the model of the surroundings, to communicate with the person (P) and/or to generate control signals for the actuator (303, 304, 401).

11. System according to any of the preceding claims, wherein information about the surroundings of the person (P) is transmitted by way of the actuator (303, 304, 401).

12. System according to any of the preceding claims, wherein the computing unit is further designed to determine, on the basis of a target position of the person (P) and the model of the surroundings, a preset path for reaching the target position and to generate control signals for the actuator (303, 304, 401) on the basis of the preset path.

13. System according to Claim 12, wherein the target position originates from a navigation system, in particular from a pedestrian navigation system.

14. System according to any of the preceding claims, wherein the computing unit is further designed to determine a confidence for the moving objects and the stationary objects in the classification of the moving objects and the stationary objects.

15. Computer-implemented method for assisting a person (P) with visual impairments, including the following method steps:
- capturing surroundings of the person (P) by means of an optical sensor (301);
- generating a three-dimensional model of the surroundings;
- distinguishing between moving objects, stationary objects and scene elements in the model of the surroundings;
- classifying the moving objects and the stationary objects;
- determining the movement trajectories of the moving objects relative to the stationary objects in the model of the surroundings;
- determining the position and the movement trajectory of the person (P) relative to the stationary objects in the model of the surroundings;
- predicting interactions on the basis of a relative movement between firstly the person (P) and secondly the moving objects, the stationary objects and scene elements;
- **characterized in that** a priority attribute is assigned to the objects, wherein either the highest priority attribute is assigned to the object predicted to have the next upcoming interaction or the object that is within a line of sight of the person (P) is assigned a higher priority attribute than an object that is away from the line of sight; and
- a control signal for a tactile and/or acoustic actuator (303, 304, 401) is generated on the basis of the predicted interactions for the object(s) with the highest priority attribute(s).

## Revendications

1. Système portable (1) permettant d'aider une personne (P) ayant des troubles visuels, comportant :
- au moins un capteur optique (301) qui est porté sur la tête de la personne (P) et qui est conçu pour détecter optiquement un environnement de la personne (P) ;
- au moins un actionneur (303, 304, 401) qui est conçu pour fournir une rétroaction tactile et/ou acoustique à la personne (P) en réponse à des signaux de commande ;
- une unité de calcul (10) qui est en liaison fonctionnelle avec le capteur (301) et l'actionneur (303, 304, 401) et qui est conçue :
o pour générer un modèle d'environnement tridimensionnel à partir de l'environnement détecté ;
o pour distinguer, dans le modèle d'environnement, des objets mobiles, des objets immobiles et des éléments de scène ;
o pour classifier les objets mobiles et les objets immobiles ;
o pour déterminer des trajectoires de mouvement des objets mobiles par rapport aux objets immobiles du modèle d'environnement ;
o pour déterminer la position de la personne (P) et une trajectoire de mouvement de la personne par rapport aux objets immobiles du modèle d'environnement ; et
o pour prédire des interactions sur la base d'un mouvement relatif entre la personne (P) d'une part et les objets mobiles, les objets immobiles et les éléments de scène d'autre part ;
**caractérisé en ce que** l'unité de calcul (10) est en outre conçue :
o pour affecter un attribut de priorité aux objets, l'attribut de priorité le plus élevé étant affecté à l'objet pour lequel une interaction chronologiquement la plus proche a été prédite ou un attribut de priorité plus élevé étant affecté à un objet qui se trouve dans un champ de vision de la personne (P) qu'à un objet qui se trouve en dehors du champ de vision ; et
o pour générer les signaux de commande pour l'actionneur (303, 304, 401) sur la base des interactions prédites pour le ou les objets ayant le/les attribut(s) de priorité le/les plus élevé(s).

2. Système selon l'une quelconque des revendications précédentes, dans lequel l'unité de calcul (10) est en outre conçue :
o pour affecter un attribut de scène au modèle d'environnement ; et
o pour générer en outre les signaux de commande pour l'actionneur (303, 304, 401) sur la base de l'attribut de scène.

3. Système selon l'une quelconque des revendications précédentes, comportant en outre un capteur acoustique (302) qui est conçu pour détecter acoustiquement l'environnement de la personne (P), et
dans lequel l'unité de calcul (10) est conçue pour générer le modèle d'environnement, l'attribut de priorité et, s'il y a référence à la revendication 2, l'attribut de scène à partir de l'environnement détecté par le capteur optique (301) et le capteur acoustique (302).

4. Système selon l'une quelconque des revendications précédentes, comportant au moins un actionneur tactile (303, 401) et au moins un actionneur acoustique (304), dans lequel l'unité de calcul (10) est conçue pour générer des signaux de commande pour l'actionneur tactile (303, 401) et/ou l'actionneur acoustique (304) en fonction d'interactions prédites.

5. Système selon l'une quelconque des revendications précédentes, comportant un premier actionneur tactile qui est conçu sous la forme d'un afficheur frontal (303) intégré dans un bandeau frontal (30) portable par la personne (P), et un deuxième actionneur tactile qui est conçu sous la forme d'un afficheur d'avant-bras (401) intégré dans un bracelet (40) portable par la personne et/ou sous la forme d'un actionneur directionnel intégré dans le bracelet (40) portable.

6. Système selon la revendication 5, dans lequel l'unité de calcul (10) est conçue pour générer les signaux de commande pour l'afficheur d'avant-bras (401) et/ou l'actionneur directionnel sur la base d'une distance et/ou d'une position spatiale entre l'un des objets d'une part et le bracelet (40), un bras de la personne (P) ou une main de la personne (P) d'autre part.

7. Système selon l'une quelconque des revendications précédentes, dans lequel l'unité de calcul (10) est conçue pour effectuer la distinction des objets, la classification des objets, la prédiction des interactions et/ou la génération des signaux de commande pour l'actionneur (303, 304, 401) à l'aide d'un ou de plusieurs procédés d'apprentissage automatique.

8. Système selon l'une quelconque des revendications précédentes, dans lequel l'unité de calcul (10) est conçue pour générer des signaux de commande identiques pour des objets qui sont classifiés dans la même classe d'objets.

9. Système selon l'une quelconque des revendications précédentes, dans lequel le capteur optique (301) est conçu sous la forme d'un système de caméra 3D, en particulier sous la forme d'un système de caméra 3D intégré dans un casque (30) portable par la personne.

10. Système selon l'une quelconque des revendications précédentes, comportant en outre une unité d'accès à distance (50) qui est conçue pour accéder au modèle d'environnement, pour communiquer avec la personne (P) et/ou pour générer des signaux de commande pour l'actionneur (303, 304, 401).

11. Système selon l'une quelconque des revendications précédentes, dans lequel des informations concernant l'environnement de la personne (P) sont transmises par l'intermédiaire de l'actionneur (303, 304, 401).

12. Système selon l'une quelconque des revendications précédentes, dans lequel l'unité de calcul est en outre conçue pour déterminer, sur la base d'une position cible de la personne (P) et du modèle d'environnement, une consigne de trajectoire pour atteindre la position cible et pour générer, sur la base de la consigne de trajectoire, des signaux de commande pour l'actionneur (303, 304, 401).

13. Système selon la revendication 12, dans lequel la position cible provient d'un système de navigation, en particulier d'un système de navigation pour piétons.

14. Système selon l'une quelconque des revendications précédentes, dans lequel l'unité de calcul est en outre conçue pour déterminer une confiance concernant les objets mobiles et les objets immobiles lors de la classification des objets mobiles et des objets immobiles.

15. Procédé mis en œuvre par ordinateur permettant d'aider une personne (P) ayant des troubles visuels, comprenant les étapes de procédé suivantes :
- détecter un environnement de la personne (P) au moyen d'un capteur optique (301) ;
- générer un modèle d'environnement tridimensionnel ;
- distinguer, dans le modèle d'environnement, des objets mobiles, des objets immobiles et des éléments de scène ;
- classifier les objets mobiles et les objets immobiles ;
- déterminer des trajectoires de mouvement des objets mobiles par rapport aux objets immobiles du modèle d'environnement ;
- déterminer la position et la trajectoire de mouvement de la personne (P) par rapport aux objets immobiles du modèle d'environnement ;
- prédire des interactions sur la base d'un mouvement relatif entre la personne (P) d'une part et les objets mobiles, les objets immobiles et les éléments de scène d'autre part ;
- **caractérisé en ce qu'**un attribut de priorité est affecté aux objets, l'attribut de priorité le plus élevé étant affecté à l'objet pour lequel une interaction chronologiquement la plus proche a été prédite ou un attribut de priorité plus élevé étant affecté à l'objet qui se trouve dans un champ de vision de la personne (P) qu'à un objet qui se trouve en dehors du champ de vision ; et
- **en ce qu'**un signal de commande pour un actionneur tactile et/ou acoustique (303, 304, 401) est généré sur la base des interactions prédites pour le ou les objets ayant le/les attribut(s) de priorité le/les plus élevé(s).
